# EUROPEAN PATENT SPECIFICATION

(11) **EP 3 794 553 B1**
(45) Date of publication and mention of the grant of the patent: **06.07.2022**
(21) Application number: 19728324.5
(22) Date of filing: 16.05.2019
(51) Int. Cl.: G06T 7/33

(54) **MULTI-MODAL IMAGE REGISTRATION**
MULTIMODALE BILDREGISTRIERUNG
SUPERPOSITION D'IMAGES MULTIMODALES

(30) Priority: 18.05.2018 US 201862673153 P
(43) Date of publication of application: 24.03.2021
(73) Proprietor: Koninklijke Philips N.V., 5656 AG Eindhoven (NL)
(72) Inventor: KRUECKER, Jochen, 5656 AE Eindhoven (NL); CHEN, Alvin, 5656 AE Eindhoven (NL)
(74) Representative: Philips Intellectual Property & Standards
(86) International application number: PCT/EP2019/062709
(87) International publication number: WO 2019/219861

(56) References cited:
- US-A1- 2017 325 785
- Jhimli Mitra: "Multimodal Image Registration Applied to Magnetic Resonance and Ultrasound Prostatic Images Multimodal Image Registration Applied to Magnetic Resonance and Ultrasound Prostatic Images.", , 1 January 2012 (2012-01-01), XP055610638, Retrieved from the Internet: URL:https://tel.archives-ouvertes.fr/docs/ 00/78/60/32/PDF/tesi-2.pdf [retrieved on 2019-08-02]
- SOUMYA GHOSE ET AL: "A hybrid framework of multiple active appearance models and global registration for 3D prostate segmentation in MRI", PROCEEDINGS SPIE 7513, 2009 INTERNATIONAL CONFERENCE ON OPTICAL INSTRUMENTS AND TECHNOLOGY, vol. 8314, 10 February 2012 (2012-02-10), page 83140S, XP055610639, 1000 20th St. Bellingham WA 98225-6705 USA ISSN: 0277-786X, DOI: 10.1117/12.911253 ISBN: 978-1-5106-2781-9
- ANDRIY FEDOROV ET AL: "Open-source image registration for MRI-TRUS fusion-guided prostate interventions", INTERNATIONAL JOURNAL OF COMPUTER ASSISTED RADIOLOGY AND SURGERY, vol. 10, no. 6, 1 June 2015 (2015-06-01), pages 925-934, XP055610644, DE ISSN: 1861-6410, DOI: 10.1007/s11548-015-1180-7

## Description

### BACKGROUND

Interventional medical procedures are procedures in which interventional medical devices are placed inside a human body. Human bodies are subject to imaging in a variety of ways, including magnetic resonance imaging (MRI) and ultrasound. Images from the different imaging modes are sometimes fused together on a display since they can present different information useful in an interventional medical device. "Fusion imaging" requires registering images together on the same coordinate system so that common features of the images appear at the same places in the images.

Accordingly, accurate multi-modal image registration is needed for "fusion imaging" procedures, such as MRI-ultrasound fusion-guided prostate biopsy. Multi-modal image registration can be very challenging due to lack of common imaging features, such as in the case of images of the prostate in MRI and ultrasound. No accurate and robust fully automatic image registration process for this purpose is known. Instead, the burden of performing, manually correcting or verifying the image registration is on the user. For inexperienced or inadequatelytrained users, creating such multi-modal image registrations is also difficult and prone to errors, leading to potentially inaccurate multi-modal image registration and thus inaccurate fusion guidance. When used for biopsy or therapy procedures, inaccurate image registration can lead to inaccurate guidance, inaccurate sampling of the tissue or even inaccurate treatment.

To add to the complexity of the technologies used in interventional medical procedures, electromagnetic tracking is used to track an ultrasound probe in a space that includes the ultrasound probe and the portions of the human body subjected to the interventional medical procedures. Moreover, in recent years, image segmentation has been applied to 2-dimensional and 3-dimensional images and image volumes, to provide views of lines, planes and other shapes where the images and image volumes are divided into structures such as organs.

A Doctoral Thesis by Mitra, J., entitled "Multimodal Image Registration applied to Magnetic Resonance and Ultrasound Prostatic Images", Signal and Image processing, Université de Bourgogne, 2012, relates to a search for a registration method with good accuracy that would facilitate sampling of prostate tissues during transrectal ultrasound guided needle biopsy.

### SUMMARY

According to an aspect of the present disclosure, a controller for registering a magnetic resonance imaging (MRI) image to a tracking space that includes a tracked ultrasound probe and an organ captured in an ultrasound image generated by the ultrasound probe, includes a memory that stores instructions; and a processor that executes the instructions. When executed by the processor, the instructions cause the controller to execute a process that includes obtaining 2-dimensional coordinates of a midsagittal cut through a segmentation of the organ based on an intersection of a 3-dimensional segmented magnetic resonance imaging volume that includes the segmentation of the organ and a midsagittal plane through the organ. The process executed by the controller also includes controlling generation of a 3-dimensional ultrasound volume; segmenting the 3-dimensional ultrasound volume to obtain a segmented 3-dimensional ultrasound volume; and identifying the midsagittal plane of the organ in the segmented 3-dimensional ultrasound volume to identify an ultrasound midsagittal plane. The process executed by the controller also includes generating, using a tracked ultrasound probe, a tracking position in the tracking space of an ultrasound image of the midsagittal plane of the organ; and registering a 2-dimensional segmented ultrasound representation of the midsagittal plane of the organ provided by the ultrasound midsagittal plane to a 2-dimensional segmented magnetic resonance imaging representation of the midsagittal plane of the organ. The process executed by the controller further includes generating a registration of the 3-dimensional magnetic resonance imaging volume to the tracking space based on the 2-dimensional coordinates of the midsagittal plane of the segmentation of the organ, the image registration of the midsagittal plane of the organ, and the tracking position in the tracking position in the tracking space of the ultrasound image of the midsagittal plane.

According to another aspect of the present disclosure, a method for registering a magnetic resonance imaging (MRI) image to a tracking space that includes a tracked ultrasound probe and an organ captured in an ultrasound image generated by the ultrasound probe, includes obtaining 2-dimensional coordinates of a midsagittal plane cut through a segmentation of the organ based on an intersection of a 3-dimensional segmented magnetic resonance imaging volume that includes the segmentation of the organ and a 2-dimensional segmented magnetic resonance imaging representation of a midsagittal plane of the organ. The method also includes controlling generation of a 3-dimensional ultrasound volume; segmenting the 3-dimensional ultrasound volume to obtain a segmented 3-dimensional ultrasound volume; and identifying the midsagittal plane of the organ in the segmented 3-dimensional ultrasound volume to identify an ultrasound midsagittal plane. The method also includes generating, using a tracked ultrasound probe, a tracking position in the tracking space of an ultrasound image of a midsagittal plane of the organ; and registering, by a processor of a controller that includes the processor and a memory, a 2-dimensional segmented ultrasound representation of the midsagittal plane of the organ provided by the ultrasound midsagittal plane to a 2-dimensional segmented magnetic resonance imaging representation of the midsagittal plane to obtain an image registration of the midsagittal plane of the organ. The method further includes generating, by the processor, a registration of the 3-dimensional magnetic resonance imaging volume to the tracking space based on the 2-dimensional coordinates of the midsagittal plane of the organ, the image registration of the midsagittal plane of the organ, and the tracking position in the tracking space of the ultrasound image of the midsagittal plane.

According to yet another aspect of the present disclosure, a system for registering a magnetic resonance imaging (MRI) image to a tracking space that includes a tracked ultrasound probe and an organ captured in an ultrasound image generated by the ultrasound probe, is defined in claim 13.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 illustrates a process for multi-modal image registration, in accordance with a representative embodiment.
FIG. 2A illustrates geometry of a segmented magnetic resonance imaging volume used in multi-modal image registration, in accordance with a representative embodiment.
FIG. 2B illustrates geometry of a segmented 3-dimensional ultrasound volume (3-D ultrasound volume), geometry of a 2-dimensional ultrasound image (2-D ultrasound image) obtained in the space of the segmented 3-D ultrasound volume, and image registration of the 2-D ultrasound image to the segmented 3-D ultrasound volume, as used in multi-modal image registration, in accordance with a representative embodiment.
FIG. 3 illustrates another process for multi-modal image registration, in accordance with a representative embodiment.
FIG. 4 illustrates a system for multi-modal image registration, in accordance with a representative embodiment.
FIG. 5 illustrates another process for multi-modal image registration, in accordance with a representative embodiment.
FIG. 6 illustrates a general computer system, on which a method of multi-modal image registration can be implemented, in accordance with a representative embodiment.

### DETAILED DESCRIPTION

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only, and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only, and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In view of the foregoing, the present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

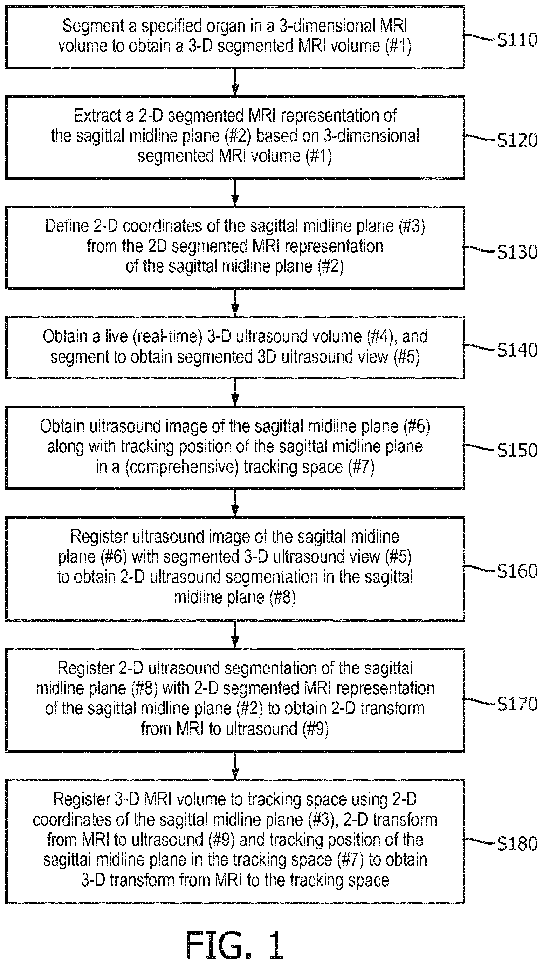

FIG. 1 illustrates a process for multi-modal image registration, in accordance with a representative embodiment.

In the description that follows, magnetic resonance imaging may be referred to as the acronyms MRI or as MR. Ultrasound may be referred to as the acronym US. Electromagnetic may be referred to as the acronym EM. Any of these acronyms may be used interchangeably with the underlying terms in the specification and Figures.

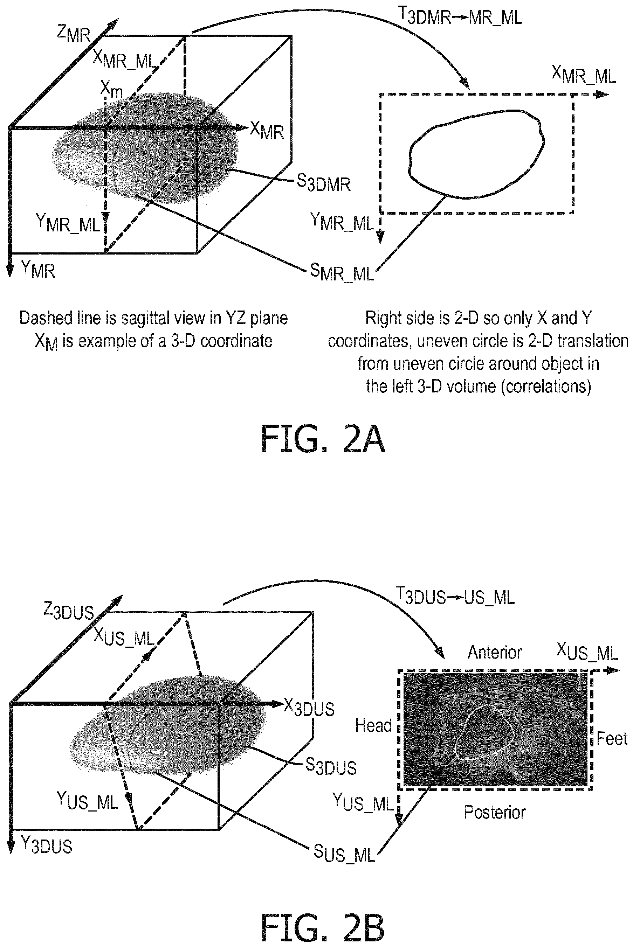

In FIG. 1, the process begins at S110 when a specified organ (e.g., a prostate) in a 3-D MRI volume is segmented to obtain a 3-D segmented MRI volume. The segmentation is a representation of the surface of the organ, and consists for example of a set of points in 3-D MRI coordinates on the surface of the organ, and triangular plane segments defined by connecting neighboring groups of 3 points, such that the entire organ surface is covered by a mesh of non-intersecting triangular planes (see e.g. Figure 2A, left side) The 3-D MRI volume may be obtained prior to an interventional medical procedure, including at a different place and on a different date.

That is, at S110, a prostate in a 3-D MRI image I_{3DMRI} may be segmented to yield the prostate 3-D MRI segmentation S_{3DMRI}. The 3-D MRI coordinate system may be defined such that an axial view of the organ corresponds to xy planes, and a sagittal view of the organ corresponds to yz planes in the volume.

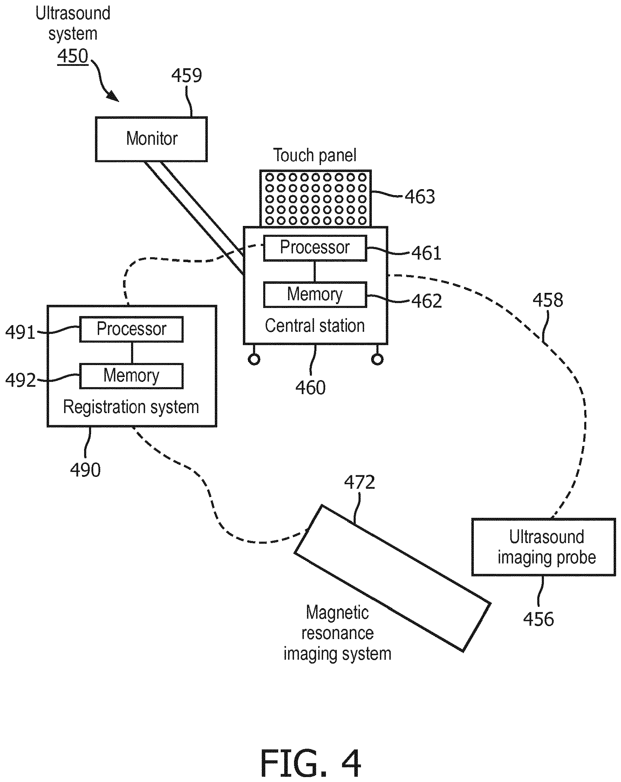

As a brief explanation of MRI, a magnetic resonance imaging system 472 shown in FIG. 4 uses a variable sequence in a transmit stage to selectively deliver a B1 field to a subject via radio frequency (RF) coils. In a receive stage, the hydrogen atoms that are stimulated by the B1 field return to an original position (i.e., the position before the selective delivery of the B1 field) and emanate a weak radio frequency signal which can be picked up by the local coils (local radio frequency coils on or near the body of the subject) and used to produce images. The magnetic resonance imaging information includes the information from the weak radio frequency signals that are detected by the local coils placed specifically to pick up the weak radio frequency signals from the hydrogen atoms of the human body.

At S120, a 2-D segmented MRI representation of a midsagittal plane is extracted from the 3-D segmented MRI volume. A midsagittal plane is an anatomical plane which divides the body or an organ into right and left parts. The plane may be in the center of the body and splits the body into two halves. In FIG. 2A and FIG. 2B explained below, the dashed lines labelled X_{MR_ML}, Y_{MR_ML} in FIG. 2A and X_{US_ML} and Y_{US_ML} in FIG. 2B define the midsagittal plane for the segmented prostate shown in 3-D volumes. Midsagittal planes are commonly used reference planes in medical imaging. Medical instruments such as MRI systems may be preset to show views for midsagittal planes. Physicians are accustomed to obtaining midsagittal view planes with freehand ultrasound.

As shown in FIG. 2A on the left, the midsagittal plane position *xₘ* in S_{3DMR}, i.e. the x-coordinate of the *yz* MRI section that cuts through the center of the prostate segmentation, can be determined. The midsagittal plane position *xₘ* in S_{3DMR} can be determined, for example, by computing the x-coordinate of the centroid of all points on the surface of S_{3DMR}. Alternatively, *xₘ* can be calculated as the midplane of the bounding box around S_{3DMR} along the x axis. Of course, *xₘ* may also be determined manually. Using *xₘ*, the transformation from the 3DMRI coordinate system to the coordinate system of the MRI midsagittal plane can be determined, i.e. T_{3DMR→MR_ML}. Given the coordinate convention defined at S130, this transformation consists of a 90-degree rotation around the y-axis, followed by a translation along the z axis by *xₘ*, as shown in FIG. 2A on the right.

As shown in FIG. 2A, the midsagittal MRI segmentation S_{MR_ML} can then be determined by computing the intersection of S_{3DMRI} with the yz plane at the x-position *xₘ*.

At S130, 2-D coordinates of the MRI midsagittal plane are defined from the 2-D segmented MRI representation of the midsagittal plane. This is specifically shown in FIG. 2A in the left box, in that the dashed lines delineate the midsagittal plane through the prostate segmentation, and 2-D coordinates of such a midsagittal plane are readily derived from the original information of the 3-D segmented MRI volume.

At S140, a 3-D ultrasound volume is obtained, and then segmented to obtain a segmented 3-D ultrasound view. The 3-D ultrasound volume does not have to be obtained live (i.e., in realtime), but can also be reconstructed from an acquisition of a set of (tracked) 2-D ultrasound planes as the ultrasound probe is swept across the organ. The 3-D ultrasound volume is typically obtained on the same day as (i.e., close in time to) the interventional medical procedure, such as at the beginning of the interventional medical procedure.

The 3-D ultrasound volume may be obtained during an interventional medical procedure, and then used as an input to the processes described herein in order to ultimately register the original 3-D segmented MRI volume with the tracking space used to track the ultrasound probe that was used to obtain the 3-D ultrasound volume. As an example, at the beginning or during an interventional medical procedure, a tracked 3-D ultrasound (3DUS) image or reconstruction I_{3DUS} of the prostate can be obtained. The tracked 3-D ultrasound reconstruction I_{3DUS} can be obtained by reconstructing into a volume a series of spatially tracked 2-dimensional ultrasound images (2-D ultrasound images) obtained while sweeping an ultrasound imaging probe across the prostate. The segmentation of I_{3DUS} yields S_{3DUS}

At S150, an ultrasound image of the midsagittal plane is acquired. The ultrasound image may be obtained such as by the ultrasound probe being controlled automatically or based on input from a user. For example, a user may be asked to specifically attempt to position the ultrasound probe such that the 2D image acquired by the probe is the midsagittal plane of the organ. As an example, an operator may be instructed to position the ultrasound probe for acquisition of a midsagittal image I_{US_ML} along the midline of the prostate, and acquire the corresponding tracked spatial pose of the ultrasound probe in the tracking space. The midsagittal plane in the tracking coordinate system corresponds to the plane *xy*_{US_ML} in Figure 2B on the left. The tracking pose of I_{US_ML} can be recorded, for example, in the form of the coordinate transformation from the 2DUS image coordinate system to the electromagnetic tracking coordinate system, T_{US→EM}.

At S160, the ultrasound image of the midsagittal plane is registered with the segmented 3-D ultrasound view to obtain a 2-D ultrasound segmentation of the midsagittal plane. For example, the midsagittal image I_{US_ML} can be automatically registered to the 3DUS segmented image volume I_{3DUS}. The intersection of I_{US_ML} with the prostate segmentation S_{3DUS} can then be computed. This intersection produces the 2-D segmentation of the prostate S_{US_ML} in I_{US_ML}, as shown on the right in FIG. 2B. The result is a more accurate representation of the midsagittal ultrasound view than simply taking the midsagittal plane of the 3DUS, because the 3DUS volume (unlike the MRI volume) is not necessarily acquired with a specific alignment of the prostate to the volume coordinate axes.

To aid the I_{US_ML} to I_{3DUS} image registration, a partial sweep I_{3DUS_ML} may be obtained starting from the midsagittal plane *xy*_{US_ML} and moving approximately in the perpendicular direction. Compared to using only a single midsagittal image I_{US_ML}, the increased spatial information contained in the partial sweep may result in a higher image registration accuracy.

At S170, the 2-D ultrasound segmentation of the midsagittal plane from S 160 is registered with the 2-D segmented MRI representation of the midsagittal plane from S120 to obtain a 2-D transform from MRI to ultrasound. In other words, a 2-D image registration is performed between S_{US_ML} and S_{MR_ML}, i.e. the midsagittal segmentations in ultrasound and MRI respectively, yielding the transformation T_{MRI→US}. The image registration can be obtained for example using the iterative closest point algorithm (ICP algorithm) to minimize the point-to-point boundary distances between S_{US_ML} and S_{MR_ML}. Alternatively, the boundary distances between the 3DMRI and 3DUS segmentations S_{3DMRI} and S_{3DUS} may be used in the minimization, while still allowing only the in-plane transformation parameters to be updated when solving for T_{MR→US}.

Compared to a full registration in six degrees-of-freedom between S_{3DMR} and S_{3DUS}, the 2-D in-plane image registration is computationally simpler and eliminates the possibility of erroneous out-of-plane translation and rotations, which are assumed to be negligible due to the user-instructed manual positioning of the probe in the midsagittal position. The boundaries may be approximately pre-aligned prior to the image registration. For example, the boundaries can be approximated based on their centroid positions.

At S180, the original 3-D MRI volume is registered to the tracking space using 2-D coordinates of the midsagittal plane from S130, the 2-D transform from the MRI to the ultrasound at S170, and the tracking position of the midsagittal plane in the tracking space from S150. Ultimately, the result of the process in FIG. 1 is a 3-D transform from MRI images to the tracking space, so that the 3-D MRI volume is registered to the tracking space. In this way, the MRI can be jointly displayed with the ultrasound in the tracking space, with accuracy not previously obtained or obtainable. That is, a display can be controlled to display live tracked ultrasound images fused with corresponding sections of magnetic resonance imaging images in an overall electromagnetic tracking space.

The way to read the individual transformations for S180 as symbolized representations is from right-to-left concatenation, to ultimately yield the desired T_{3DMR→EM}. Notably, the ultrasound coordinate system US_ML is being equated with the 2-D ultrasound coordinate system because the user was instructed to obtain the 2DUS image in the midsagittal position. The midsagittal position is a standard clinical ultrasound view, at least for a prostate, which physicians familiar with this subject matter should be able to readily identify.

The resulting transformation T_{3DMR→EM} can be used for fusion imaging display of live tracked ultrasound images with corresponding sections of the MRI image. This can be done, for example, by obtaining the tracking position T_{US→EM} of any live ultrasound image, and computing T_{US→3DMR} = (T_{3DMR→EM})⁻¹ · T_{US→EM} to get the MRI coordinates that correspond to the current 2DUS image, where (·)⁻¹ indicates the transform inversion.

FIG. 2A illustrates geometry of a segmented magnetic resonance imaging volume used in multi-modal image registration, in accordance with a representative embodiment.

FIG. 2A shows the extraction of the midsagittal cut through the 3DMRI prostate segmentation, yielding S_{MR_ML} as isolated in 2-D on the right. The coordinate Xm shown on the left in FIG. 2A is a 3-D coordinate in an XYZ coordinate system. The dashed lines are the sagittal view in the YZ plane. The irregularly shaped object in the bounding box on the left is the segmentation of the prostate in 3-D. Notably, the solid irregular line around the representation of the prostate in 3-D on the left of FIG. 2A is the intersection of the prostate segmentation with the midsagittal plane of the prostate and correlates to the solid irregular line in 2-D on the right of FIG. 2A. That is, the right side of FIG. 2A shows the 2-D representation of the segmented midsagittal plane of the prostate in the MRI

FIG. 2B illustrates geometry of a segmented 3-D ultrasound volume, geometry of a 2-D ultrasound image obtained in the same space as the 3-D ultrasound volume, and image registration of the 2-D ultrasound image to the segmented 3-D ultrasound volume, as used in multi-modal image registration, in accordance with a representative embodiment.

FIG. 2B shows the 2-D ultrasound image obtained in the midsagittal view I_{US_ML} registered into the 3-D ultrasound volume on the left. The corresponding intersection with the 3-D ultrasound volume is transformed into the I_{US_ML} coordinates. In other words, the 3-D model on the left in FIG. 2B correlates again to the 2-D image on the right.

Incidentally, in the image on the right in FIG. 2B, the anterior side is the top, the posterior side is the bottom, the head is to the left, and the feet are to the right. Additionally, the solid irregular line around the representation of the prostate on the left of FIG. 2B correlates to the solid irregular line on the right of FIG. 2B. Specifically, the solid irregular line in FIG. 2B is the intersection of the midsagittal plane with the organ segmentation as shown in 3D MRI coordinates on the left.

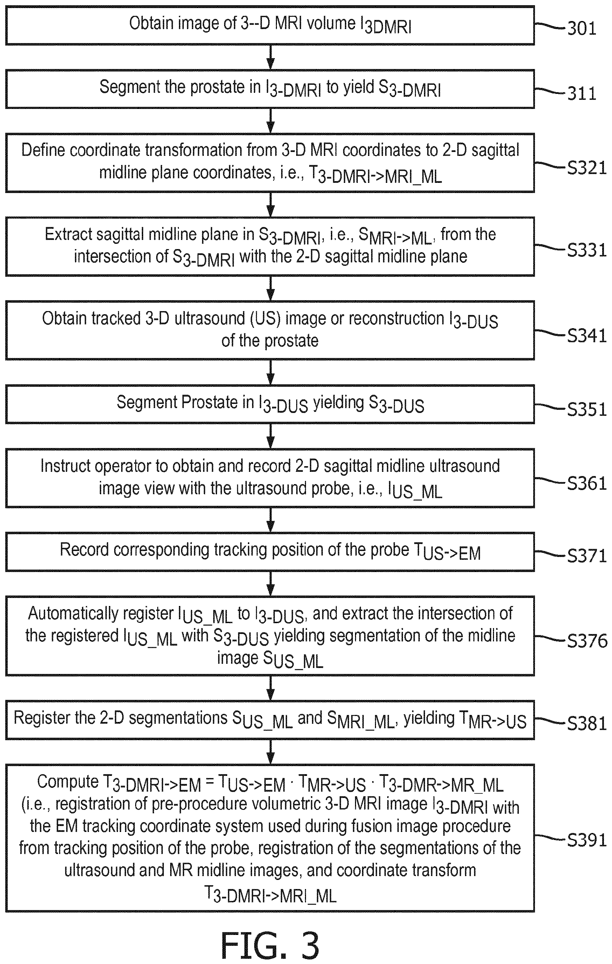

FIG. 3 illustrates another process for multi-modal image registration, in accordance with a representative embodiment.

FIG. 3 shows and describes an embodiment of elements of the specific case of MRI-ultrasound image registration for prostate fusion biopsy with electromagnetic tracking of the ultrasound probe. In other words, three separate visualizations are combined in the embodiment of FIG. 3., i.e., MRI that includes the prostate, ultrasound that includes the prostate, and electromagnetic tracking in a 3-dimensional space that includes the ultrasound imaging probe and the prostate. The features shown in and described with respect to FIG. 3 are applicable to other organs, imaging modalities and procedures.

In FIG. 3, the objective is to compute the image registration of a pre-acquired MRI volume image (I_{3-DMRI}) with the electromagnetic tracking coordinate system used during the fusion imaging procedure, i.e. T_{3-DMRI}→_{EM.}

In a first step or process at S301, an image of a 3-D MRI volume of the prostate is obtained as I_{3-DMRI}. In a second step or process at S311, the image of the 3-D MRI volume of the prostate I_{3-DMRI} is segmented, to yield the prostate 3-D MRI segmentation S_{3-DMRI}.

In a third step or process at S321, a coordinate transformation from 3-D MRI to the 2-D midsagittal plane coordinates is defined, i.e., T_{3-DMRI}→_{MRI_ML}. In a fourth step or process at S331, the midsagittal plane in the MRI segmentation S_{3-DMRI} is extracted, i.e., S_{MRI_ML}. The midsagittal plane in the MRI segmentation is the intersection of S_{3-DMRI} with the 2-D midsagittal plane.

In a fifth step or process at S341, a tracked 3-D ultrasound volume or reconstruction of the prostate is obtained, i.e., I_{3DUS}. In a sixth step or process at S351, the prostate in the 3-D ultrasound volume I_{3DUS} is segmented to yield S_{3DUS}.

In a seventh step or process at S361, the operator is instructed to obtain and record a midsagittal ultrasound image I_{US_ML} of the prostate with the ultrasound probe. In an eighth step or process at S371, the tracking position T_{US→EM} of the ultrasound probe is recorded in the electromagnetic tracking space.

In a ninth step or process at S376, the midsagittal ultrasound image I_{US_ML} is automatically registered with/to the 3-D ultrasound volume I_{3DUS}. The intersection of I_{US_ML} with the prostate segmentation S_{3DUS} is extracted by computation to produce the 2-D segmentation of the prostate S_{US_ML} in the midsagittal image.

In a tenth step or process at S381, a 2-D image registration is performed between the segmentation of the ultrasound midsagittal plane S_{US_ML} and the segmentation of the magnetic resonance imaging midsagittal plane S_{MRI_ML}. The 2-D image registration in the tenth step or process may be performed using, for example, an iterative closest point (ICP) algorithm. The result of the tenth step or process is the transformation of the magnetic resonance imaging coordinates to ultrasound coordinates T_{MRI}→_{US}.

In an eleventh step or process at S391, a series of transformations results in the 3-D magnetic resonance imaging being registered to the electromagnetic tracking, i.e., T_{3-DMRI}→_{EM.} The transformations are from the 3-D magnetic resonance imaging to the magnetic resonance imaging midsagittal plane, then from the magnetic resonance imaging midsagittal plane to ultrasound midsagittal plane, and then from ultrasound midsagittal plane to the electromagnetic tracking field, i.e., T_{3-DMRI}→_{MRI_ML}, _{TMRI→US} and T_{US→EM}, to yield the desired T_{3-DMRI}→_{EM}. That is, the end result at S391 is the image registration of the pre-acquired MRI volume 3-DMRI with the electromagnetic tracking coordinate system used during the fusion imaging procedure, i.e. T_{3-DMRI}→_{EM.}

FIG. 4 illustrates a system for multi-modal image registration, in accordance with a representative embodiment.

In FIG. 4, an ultrasound system 450 includes a central station 460 with a processor 461 and memory 462, a touch panel 463, a monitor 459, and an ultrasound imaging probe 456 connected to the central station 460 by a data connection 458 (e.g., a wired or wireless data connection). Though the multi-modal image registration described herein will typically or at least often involve an interventional medical process, no interventional medical device is shown in FIG. 4, as the multi-modal image registration is more concerned with registering images from different modes than anything particular to an interventional medical device.

A magnetic resonance imaging system 472 is also shown. To be clear, MRI images used in multi-modal image registration may be provided from a different time and a different place than the ultrasound system 450, in which case the magnetic resonance imaging system 472 is not necessarily in the same place as the ultrasound system 450. In FIG. 4, the magnetic resonance imaging system 472 is shown together with the ultrasound system 450, and images from the MRI mode and the ultrasound mode are provided to a registration system 490 that includes a processor 491 and a memory 492. In the context of multi-modal image registration, the registration system 490 may be considered a controller that controls a process for registering images from multiple modes as described herein. In other words, the registration system 490 may be a controller that performs or otherwise controls performance of functionality and processes described herein. A registration system 490 may be a stand-alone system, or may be implemented in a modified electronic system such as an ultrasound system used in interventional medical procedures.

The registration system 490 includes a processor 491 and a memory 492. The registration system 490 receives data from the magnetic resonance imaging system 472, either directly or indirectly such as over a network or from a computer readable medium. The registration system 490 performs processes described herein by, for example, the processor 491 executing instructions in the memory 492. However, the registration system 490 may also be implemented in or by the central station 460, or in any other mechanism. The combination of the processor 491 and memory 492, whether in the registration system 490 or in another configuration, may be considered a "controller" as the term is used herein.

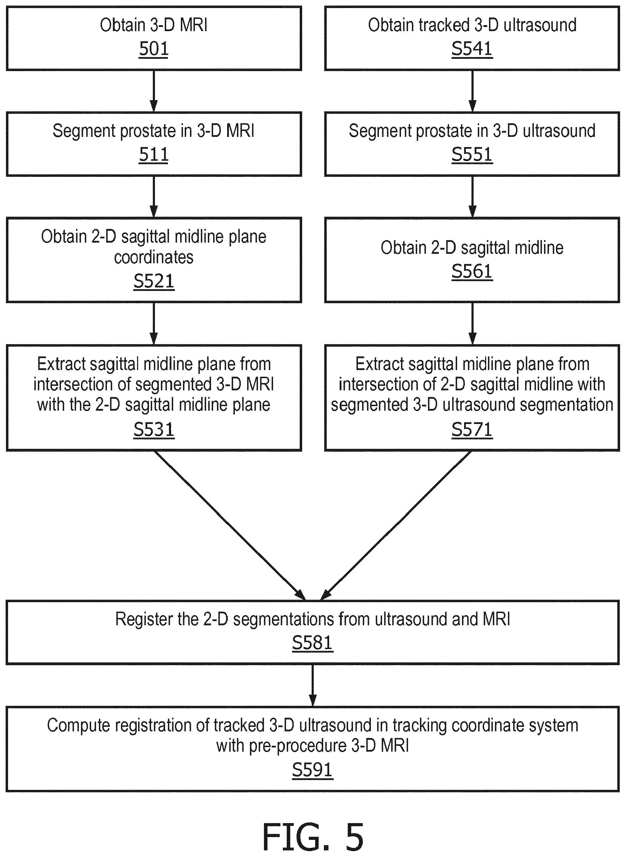

FIG. 5 illustrates another process for multi-modal image registration, in accordance with a representative embodiment.

In FIG. 5, the left side initially shows MRI processes, and the right side initially shows ultrasound processes. This visualization reflects that the different processes may be performed in parallel, or at entirely different times and in different places.

At S501, a 3-D MRI volume is acquired. At S511, a prostate in the 3-D MRI volume is segmented. At S521, the 2-D midsagittal plane coordinates are obtained, and at S531 the midsagittal segmentation plane is extracted from the intersection of the segmented 3-D MRI with the 2-D midsagittal plane.

At S541, a tracked 3-D ultrasound is obtained. At S551, the prostate in the tracked 3-D ultrasound is segmented. At S561, the 2-D midsagittal plane of the 3D ultrasound is obtained, and at S571, the midsagittal segmentation plane is extracted from the intersection of the 2-D midsagittal plane with the segmented 3-D ultrasound segmentation.

At S581, the 2-D segmentations from ultrasound and MRI are registered. At S591, the tracked 3-D ultrasound in the tracking coordinate system is registered with the pre-procedure 3-D MRI. As a result, both the pre-procedure MRI and the ultrasound imagery can be displayed in the same coordinate space. Specifically, the pre-procedure MRI and the ultrasound can be displayed in the tracking space provided initially for the ultrasound to track the ultrasound probe.

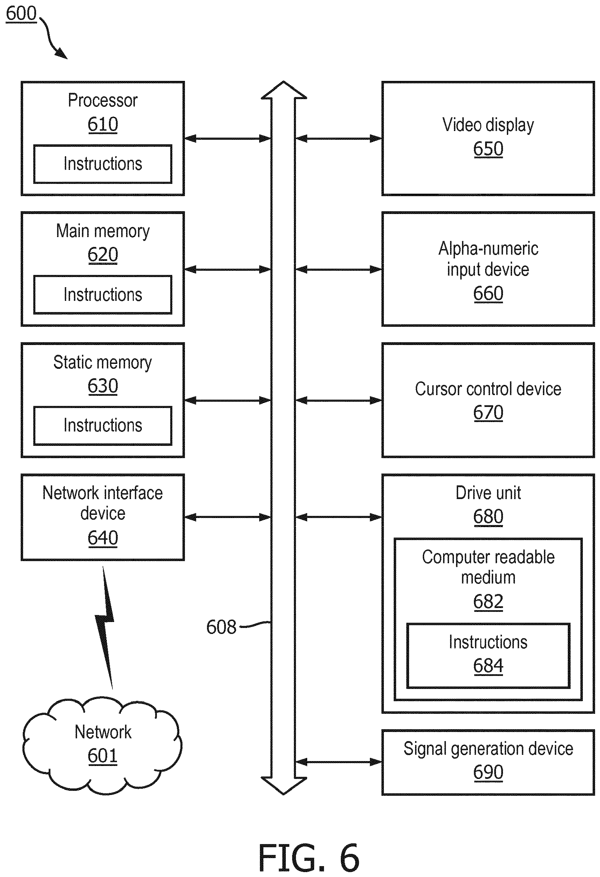

FIG. 6 illustrates a general computer system, on which a method of multi-modal image registration can be implemented, in accordance with a representative embodiment.

The computer system 600 can include a set of instructions that can be executed to cause the computer system 600 to perform any one or more of the methods or computer based functions disclosed herein. The computer system 600 may operate as a standalone device or may be connected, for example, using a network 601, to other computer systems or peripheral devices. Any or all of the elements and characteristics of the computer system 600 in FIG. 6 may be representative of elements and characteristics of the central station 460, the registration system 490, the ultrasound imaging probe 456, the ultrasound system 450, or other similar devices and systems that can include a controller and perform the processes described herein.

In a networked deployment, the computer system 600 may operate in the capacity of a client in a server-client user network environment. The computer system 600 can also be fully or partially implemented as or incorporated into various devices, such as a control station, imaging probe, passive ultrasound sensor, stationary computer, a mobile computer, a personal computer (PC), or any other machine capable of executing a set of instructions (sequential or otherwise) that specify actions to be taken by that machine. The computer system 600 can be incorporated as or in a device that in turn is in an integrated system that includes additional devices. In an embodiment, the computer system 600 can be implemented using electronic devices that provide video or data communication. Further, while the computer system 600 is illustrated, the term "system" shall also be taken to include any collection of systems or sub-systems that individually or jointly execute a set, or multiple sets, of instructions to perform one or more computer functions.

As illustrated in FIG. 6, the computer system 600 includes a processor 610. A processor 610 for a computer system 600 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. Any processor described herein is an article of manufacture and/or a machine component. A processor for a computer system 600 is configured to execute software instructions to perform functions as described in the various embodiments herein. A processor for a computer system 600 may be a general-purpose processor or may be part of an application specific integrated circuit (ASIC). A processor for a computer system 600 may also be a microprocessor, a microcomputer, a processor chip, a controller, a microcontroller, a digital signal processor (DSP), a state machine, or a programmable logic device. A processor for a computer system 600 may also be a logical circuit, including a programmable gate array (PGA) such as a field programmable gate array (FPGA), or another type of circuit that includes discrete gate and/or transistor logic. A processor for a computer system 600 may be a central processing unit (CPU), a graphics processing unit (GPU), or both. Additionally, any processor described herein may include multiple processors, parallel processors, or both. Multiple processors may be included in, or coupled to, a single device or multiple devices.

Moreover, the computer system 600 includes a main memory 620 and a static memory 630 that can communicate with each other via a bus 608. Memories described herein are tangible storage mediums that can store data and executable instructions, and are non-transitory during the time instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A memory described herein is an article of manufacture and/or machine component. Memories described herein are computer-readable mediums from which data and executable instructions can be read by a computer. Memories as described herein may be random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. Memories may be volatile or non-volatile, secure and/or encrypted, unsecure and/or unencrypted.

As shown, the computer system 600 may further include a video display unit 650, such as a liquid crystal display (LCD), an organic light emitting diode (OLED), a flat panel display, a solid-state display, or a cathode ray tube (CRT). Additionally, the computer system 600 may include an input device 660, such as a keyboard/virtual keyboard or touch-sensitive input screen or speech input with speech recognition, and a cursor control device 670, such as a mouse or touch-sensitive input screen or pad. The computer system 600 can also include a disk drive unit 680, a signal generation device 690, such as a speaker or remote control, and a network interface device 640.

In an embodiment, as depicted in FIG. 6, the disk drive unit 680 may include a computer-readable medium 682 in which one or more sets of instructions 684, e.g. software, can be embedded. Sets of instructions 684 can be read from the computer-readable medium 682. Further, the instructions 684, when executed by a processor, can be used to perform one or more of the methods and processes as described herein. In an embodiment, the instructions 684 may reside completely, or at least partially, within the main memory 620, the static memory 630, and/or within the processor 610 during execution by the computer system 600.

In an alternative embodiment, dedicated hardware implementations, such as applicationspecific integrated circuits (ASICs), programmable logic arrays and other hardware components, can be constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing can be constructed to implement one or more of the methods or functionality as described herein, and a processor described herein may be used to support a virtual processing environment.

The present disclosure contemplates a computer-readable medium 682 that includes instructions 684 or receives and executes instructions 684 responsive to a propagated signal; so that a device connected to a network 601 can communicate video or data over the network 601. Further, the instructions 684 may be transmitted or received over the network 601 via the network interface device 640.

Accordingly, multi-modal image registration enables image registration of pre-procedure MRI with intra-procedure ultrasound, even with imaged features which are not common to the different modes such as when the prostate is imaged.

Although multi-modal image registration has been described with reference to several exemplary embodiments, it is understood that the words that have been used are words of description and illustration, rather than words of limitation. Changes may be made within the purview of the appended claims, as presently stated and as amended, without departing from the scope of multi-modal image registration as defined by the claims. Although multi-modal image registration has been described with reference to particular means, materials and embodiments, multi-modal image registration is not intended to be limited to the particulars disclosed; rather multi-modal image registration extends to all functionally equivalent structures, methods, and uses such as are within the scope of the appended claims.

For example, re-registration or motion compensation can be performed using features described herein, such as the latter features of the processes shown in and described for the methods of FIGs. 1, 3 and 5. Re-registration or even motion compensation can be performed when the prostate has moved for example. That is, processes described herein can include re-generating, based on determining movement of the prostate, the image registration of the 3-dimensional magnetic resonance imaging volume in the tracking space based on the 2-dimensional coordinates of the midsagittal plane of the organ, the image registration of the midsagittal plane of the organ, and the tracking position in the tracking space of the ultrasound image of the midsagittal plane.

Additionally, the 2-D MRI midsagittal plane segmentation S_{MR_ML} can be obtained directly without first obtaining S_{3DMR}, such as by estimating the x-position of the midsagittal plane through the prostate in the 3-D MRI image. This can be done by assuming the prostate is in the center of the 3-D MRI image for example. The 2-D segmentation can then be performed only in the chosen midsagittal plane of the 3-D MRI volume.

Additionally, the 2-D midsagittal ultrasound image of the prostate can be segmented directly rather than registering to the 3-D ultrasound volume and extracting the 2-D section through the 3-D segmentation.

As another alternative, if the midsagittal plane of the 3-D ultrasound is known, a 2-D image registration can be performed directly. Specifically, the midsagittal plane of 3-D ultrasound can be registered directly to the image of the ultrasound midsagittal plane in place of the 2D-to-3D image registration described in embodiments above. The midsagittal plane of 3-D ultrasound can be identified in a number of ways, including automatically, based on manual assessment of the volume, or based on specific user instructions carried out during the acquisition of the 3-D sweep.

As yet another alternative, depending on the ultrasound probe type used and the organ imaged, a different reference view other than "sagittal" could be chosen. For example, axial or coronal views can be chosen and used.

The illustrations of the embodiments described herein are intended to provide a general understanding of the structure of the various embodiments. Although embodiments discussed are related to fusion guided prostate biopsy, the invention is not so limited. In particular, the disclosure herein is generally applicable to other organs as well as to the prostate. Illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

In the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

## Claims

1. A controller for registering a magnetic resonance imaging (MRI) image to a tracking space that includes a tracked ultrasound probe and an organ captured in an ultrasound image generated by the ultrasound probe, comprising:
a memory (492, 620, 630) that stores instructions; and
a processor (491, 610) that executes the instructions,
wherein, when executed by the processor, the instructions cause the controller to execute a process comprising:
obtaining (S110, S120, S130, S331) 2-dimensional coordinates of a midsagittal cut through a segmentation of the organ based on an intersection of a 3-dimensional segmented magnetic resonance imaging volume that includes the segmentation of the organ and a midsagittal plane through the organ;
controlling (S140, S341) generation of a 3-dimensional ultrasound volume;
segmenting (S140, S351) the 3-dimensional ultrasound volume to obtain a segmented 3-dimensional ultrasound volume;
identifying (S150, S361) the midsagittal plane of the organ in the segmented 3-dimensional ultrasound volume to identify an ultrasound midsagittal plane;
generating (S150, S371), using a tracked ultrasound probe, a tracking position in the tracking space of an ultrasound image of the midsagittal plane of the organ;
registering (S170, S381) a 2-dimensional segmented ultrasound representation of the midsagittal plane of the organ provided by the ultrasound midsagittal plane to a 2-dimensional segmented magnetic resonance imaging representation of the midsagittal plane of the organ to obtain an image registration of the midsagittal plane of the organ; and
generating (S180, S391) a registration of the 3-dimensional magnetic resonance imaging volume to the tracking space based on the 2-dimensional coordinates of the midsagittal plane of the segmentation of the organ, the image registration of the midsagittal plane of the organ, and the tracking position in the tracking space of the ultrasound image of the midsagittal plane.

2. The controller of claim 1, wherein the process executed by the controller further comprises:
segmenting (311) a 3-dimensional magnetic resonance imaging volume to obtain the 3-dimensional segmented magnetic resonance imaging volume; and
identifying (S331) the midsagittal plane of the organ in the 3-dimensional segmented magnetic resonance imaging volume to obtain the 2-dimensional segmented magnetic resonance imaging representation of the midsagittal plane of the organ.

3. The controller of claim 2, wherein the process executed by the controller further comprises:
transforming coordinates of the 2-dimensional segmented magnetic resonance imaging representation of the midsagittal plane to the 2-dimensional coordinates of the midsagittal plane of the organ.

4. The controller of claim 1, wherein the midsagittal plane of the organ is identified based on input prompted from a user.

5. The controller of claim 1, wherein the process executed by the controller further comprises:
obtaining the 2-dimensional segmented magnetic resonance imaging representation of the midsagittal plane of the organ; and
registering the 2-dimensional segmented magnetic resonance imaging representation of the midsagittal plane of the organ to the ultrasound midsagittal plane.

6. The controller of claim 5, wherein the registering of the 2-dimensional segmented magnetic resonance imaging representation of the midsagittal plane of the organ to the ultrasound midsagittal plane is performed using an iterative closest point algorithm.

7. The controller of claim 1,
wherein the organ comprises a prostate.

8. The controller of claim 1,
wherein the image registration of the 3-dimensional magnetic resonance imaging volume in the tracking space is performed live during an interventional medical procedure.

9. The controller of claim 1, wherein the process executed by the controller further comprises:
generating a 3-dimensional ultrasound volume by reconstructing into a volume a series of spatially tracked 2-dimensional ultrasound images obtained while sweeping the ultrasound probe across the organ.

10. The controller of claim 1, wherein the process executed by the controller further comprises:
controlling a display to display live tracked ultrasound images fused with corresponding sections of magnetic resonance imaging images.

11. The controller of claim 1, wherein the process executed by the controller further comprises:
determining movement of the organ; and
re-generating, based on determining movement of the organ, the registration of the 3-dimensional magnetic resonance imaging volume to the tracking space based on the 2-dimensional coordinates of the midsagittal plane of the organ, the image registration of the midsagittal plane of the organ, and the tracking position in the tracking space of the ultrasound image of the midsagittal plane.

12. A method for registering a magnetic resonance imaging (MRI) image to a tracking space that includes a tracked ultrasound probe and an organ captured in an ultrasound image generated by the ultrasound probe, comprising:
obtaining 2-dimensional coordinates of a midsagittal plane cut through a segmentation of the organ based on an intersection of a 3-dimensional segmented magnetic resonance imaging volume that includes the segmentation of the organ and a 2-dimensional segmented magnetic resonance imaging representation of a midsagittal plane of the organ;
controlling generation of a 3-dimensional ultrasound volume;
segmenting the 3-dimensional ultrasound volume to obtain a segmented 3-dimensional ultrasound volume;
identifying the midsagittal plane of the organ in the segmented 3-dimensional ultrasound volume to identify an ultrasound midsagittal plane;
generating, using a tracked ultrasound probe, a tracking position in the tracking space of an ultrasound image of a midsagittal plane of the organ;
registering, by a processor of a controller that includes the processor and a memory, a 2-dimensional segmented ultrasound representation of the midsagittal plane of the organ provided by the ultrasound midsagittal plane to a 2-dimensional segmented magnetic resonance imaging representation of the midsagittal plane to obtain an image registration of the midsagittal plane of the organ; and
generating, by the processor, a registration of the 3-dimensional magnetic resonance imaging volume to the tracking space based on the 2-dimensional coordinates of the midsagittal plane of the organ, the image registration of the midsagittal plane of the organ, and the tracking position in the tracking space of the ultrasound image of the midsagittal plane.

13. A system for registering a magnetic resonance imaging (MRI) image to a tracking space that includes a tracked ultrasound probe and an organ captured in an ultrasound image generated by the ultrasound probe, comprising:
the controller of claim 1, and a tracked ultrasound probe (456);

## Patentansprüche

1. Eine Steuerung zum Erfassen eines Magnetresonanztomographie-Bilds (MRT) in einem Nachverfolgungsraum, der eine nachverfolgte Ultraschallsonde und ein Organ umfasst, das in einem von der Ultraschallsonde erzeugten Ultraschallbild erfasst wird, wobei die Steuerung Folgendes umfasst:
einen Speicher (492, 620, 630), auf dem Befehle gespeichert werden; und
einen Prozessor (491, 610), der die Befehle ausführt, wobei die vom Prozessor ausgeführten Befehle die Steuerung dazu veranlassen, einen Prozess auszuführen, der folgende Schritte umfasst:
Abrufen (S110, S120, S130, S331) der 2-dimensionalen Koordinaten eines Mediansagittalschnitts durch eine Segmentierung des Organs beruhend auf dem Schnittpunkt eines 3-dimensionalen segmentierten Magnetresonanztomographie-Volumens, das die Segmentierung des Organs umfasst, und einer Mediansagittalebene durch das Organ;
Steuern (S140, S341) des Erstellens eines 3-dimensionalen Ultraschallvolumens;
Segmentieren (S140, S351) des 3-dimensionalen Ultraschallvolumens, um ein segmentiertes 3-dimensionales Ultraschallvolumen zu erhalten;
Ermitteln (S150, S361) der Mediansagittalebene des Organs im segmentierten 3-dimensionalen Ultraschallvolumen, um eine Ultraschall-Mediansagittalebene zu ermitteln;
mithilfe einer nachverfolgten Ultraschallsonde Erstellen (S150, S371) einer Nachverfolgungsposition im Nachverfolgungsraum eines Ultraschallbilds der Mediansagittalebene des Organs;
Erfassen (S170, S381) einer 2-dimensionalen segmentierten Ultraschalldarstellung der Mediansagittalebene des Organs, die anhand der Ultraschall-Mediansagittalebene bereitgestellt wird, für eine 2-dimensionale segmentierte Magnetresonanztomographie-Darstellung der Mediansagittalebene des Organs, um eine Bilderfassung der Mediansagittalebene des Organs zu erhalten; und
Erstellen (S180, S391) einer Erfassung des 3-dimensionalen Magnetresonanztomographie-Volumens im Verfolgungsraum anhand der 2-dimensionalen Koordinaten der Mediansagittalebene der Segmentierung des Organs, der Bilderfassung der Mediansagittalebene des Organs und der Nachverfolgungsposition im Nachverfolgungsraum des Ultraschallbilds der Mediansagittalebene.

2. Die Steuerung gemäß Anspruch 1, wobei der von der Steuerung durchgeführte Prozess zudem folgende Schritte umfasst:
Segmentieren (311) eines 3-dimensionalen Magnetresonanztomographie-Volumens, um das 3-dimensionale segmentierte Magnetresonanztomographie-Volumen zu erhalten; und
Ermitteln (S331) der Mediansagittalebene des Organs im 3-dimensionalen segmentierten Magnetresonanztomographie-Volumen, um die 2-dimensionale segmentierte Magnetresonanztomographie-Darstellung der Mediansagittalebene des Organs zu erhalten.

3. Die Steuerung gemäß Anspruch 2, wobei der von der Steuerung durchgeführte Prozess zudem folgenden Schritt umfasst:
Umwandeln der Koordinaten der 2-dimensionalen segmentierten Magnetresonanztomographie-Darstellung der Mediansagittalebene in die 2-dimensionalen Koordinaten der Mediansagittalebene des Organs.

4. Die Steuerung gemäß Anspruch 1, wobei die Mediansagittalebene des Organs anhand einer von einem Benutzer veranlassten Eingabe ermittelt wird.

5. Die Steuerung gemäß Anspruch 1, wobei der von der Steuerung durchgeführte Prozess zudem folgende Schritte umfasst:
Abrufen der 2-dimensionalen segmentierten Magnetresonanztomographie-Darstellung der Mediansagittalebene des Organs; und
Erfassen der 2-dimensionalen segmentierten Magnetresonanztomographie-Darstellung der Mediansagittalebene des Organs für die Ultraschall-Mediansagittalebene.

6. Die Steuerung gemäß Anspruch 5, wobei das Erfassen der 2-dimensionalen segmentierten Magnetresonanztomographie-Darstellung der Mediansagittalebene des Organs für die Ultraschall-Mediansagittalebene mithilfe eines iterativen Nächste-Punkte-Algorithmus durchgeführt wird.

7. Die Steuerung gemäß Anspruch 1,
wobei das Organ eine Prostata umfasst.

8. Die Steuerung gemäß Anspruch 1,
wobei die Bilderfassung des 3-dimensionalen Magnetresonanztomographie-Volumens im Nachverfolgungsraum live während eines medizinischen Eingriffs durchgeführt wird.

9. Die Steuerung gemäß Anspruch 1, wobei der von der Steuerung durchgeführte Prozess zudem folgenden Schritt umfasst:
Erstellen eines 3-dimensionalen Ultraschallvolumens durch Rekonstruieren einer Reihe von in einem Volumen räumlich nachverfolgten 2-dimensionalen Ultraschallbildern, die abgerufen werden, während die Ultraschallsonde über das Organ bewegt wird.

10. Die Steuerung gemäß Anspruch 1, wobei der von der Steuerung durchgeführte Prozess zudem folgenden Schritt umfasst:
Steuern einer Anzeige zum Anzeigen der live nachverfolgten Ultraschallbilder, die mit den entsprechenden Abschnitten der Magnetresonanztomographie-Bildern zusammengeführt werden.

11. Die Steuerung gemäß Anspruch 1, wobei der von der Steuerung durchgeführte Prozess zudem folgende Schritte umfasst:
Ermitteln der Bewegung des Organs; und
beruhend auf der ermittelten Bewegung des Organs erneutes Erstellen einer Erfassung des 3-dimensionalen Magnetresonanztomographie-Volumens im Verfolgungsraum anhand der 2-dimensionalen Koordinaten der Mediansagittalebene des Organs, der Bilderfassung der Mediansagittalebene des Organs und der Nachverfolgungsposition im Nachverfolgungsraum des Ultraschallbilds der Mediansagittalebene.

12. Eine Methode zum Erfassen eines Magnetresonanztomographie-Bilds (MRT) in einem Nachverfolgungsraum, der eine nachverfolgte Ultraschallsonde und ein Organ umfasst, das in einem von der Ultraschallsonde erzeugten Ultraschallbild erfasst wird, wobei die Methode folgende Schritte umfasst:
Abrufen der 2-dimensionalen Koordinaten eines Mediansagittalebenen-Schnitts durch eine Segmentierung des Organs beruhend auf dem Schnittpunkt eines 3-dimensionalen segmentierten Magnetresonanztomographie-Volumens, das die Segmentierung des Organs umfasst, und einer 2-dimensionalen segmentierten Magnetresonanztomographie-Darstellung einer Mediansagittalebene des Organs;
Steuern des Erstellens eines 3-dimensionalen Ultraschallvolumens;
Segmentieren des 3-dimensionalen Ultraschallvolumens,
um ein segmentiertes 3-dimensionales Ultraschallvolumen zu erhalten;
Ermitteln der Mediansagittalebene des Organs im segmentierten 3-dimensionalen Ultraschallvolumen, um eine Ultraschall-Mediansagittalebene zu ermitteln;
mithilfe einer nachverfolgten Ultraschallsonde Erstellen einer Nachverfolgungsposition im Nachverfolgungsraum eines Ultraschallbilds der Mediansagittalebene des Organs;
mit dem Prozessor einer Steuerung, die den Prozessor und einen Speicher umfasst, Erfassen einer 2-dimensionalen segmentierten Ultraschalldarstellung der Mediansagittalebene des Organs, die anhand der Ultraschall-Mediansagittalebene bereitgestellt wird,
für eine 2-dimensionale segmentierte Magnetresonanztomographie-Darstellung der Mediansagittalebene, um eine Bilderfassung der Mediansagittalebene des Organs zu erhalten; und
mit dem Prozessor Erstellen einer Erfassung des 3-dimensionalen Magnetresonanztomographie-Volumens im Verfolgungsraum anhand der 2-dimensionalen Koordinaten der Mediansagittalebene des Organs, der Bilderfassung der Mediansagittalebene des Organs und der Nachverfolgungsposition im Nachverfolgungsraum des Ultraschallbilds der Mediansagittalebene.

13. Ein System zum Erfassen eines Magnetresonanzbilds (MRT) in einem Nachverfolgungsraum, der eine nachverfolgte Ultraschallsonde und ein Organ umfasst, das in einem von der Ultraschallsonde erzeugten Ultraschallbild erfasst wird, wobei das System Folgendes umfasst:
die Steuerung gemäß Anspruch 1, und
eine nachverfolgte Ultraschallsonde (456);

## Revendications

1. Contrôleur pour enregistrer une image par imagerie par résonance magnétique (IRM) dans un espace de suivi qui comprend une sonde à ultrasons suivie et un organe capturé dans une image à ultrasons générée par la sonde à ultrasons, comprenant :
une mémoire (492, 620, 630) qui stocke les instructions ; et
un processeur (491, 610) qui exécute les instructions ;
dans lequel, lorsqu'exécutées par le processeur, les instructions entraînent le contrôleur à exécuter un processus comprenant :
l'obtention (S110, S120, S130, S331) de coordonnées à 2 dimensions d'une coupe médiane à travers une segmentation de l'organe sur la base d'une intersection d'un volume d'imagerie à résonance magnétique à 3 dimensions segmenté qui comprend la segmentation de l'organe et un plan médian à travers l'organe ;
le contrôle (S140, S341) de la génération d'un volume ultrasonore à 3 dimensions ;
la segmentation (S140, S351) du volume ultrasonore à 3 dimensions pour obtenir un volume ultrasonore à 3 dimensions segmenté ;
l'identification (S150, S361) du plan médian de l'organe dans le volume ultrasonore à 3 dimensions segmenté pour identifier un plan médian ultrasonore ;
la génération (S150, S371), en utilisant une sonde à ultrasons suivie, d'une position de suivi dans l'espace de suivi d'une image à ultrasons du plan médian de l'organe ;
l'enregistrement (S170, S381) d'une représentation ultrasonore segmentée à 2 dimensions du plan médian de l'organe fournie par le plan médian ultrasonore dans une représentation d'imagerie à résonance magnétique segmentée à 2 dimensions du plan médian de l'organe pour obtenir un enregistrement d'image du plan médian de l'organe ; et
la génération (S180, S391) d'un enregistrement du volume d'imagerie à résonance magnétique à 3 dimensions dans l'espace de suivi sur la base des coordonnées à 2 dimensions du plan médian de la segmentation de l'organe, l'enregistrement d'image du plan médian de l'organe, et la position de suivi dans l'espace de suivi de l'image à ultrasons du plan médian.

2. Contrôleur selon la revendication 1, dans lequel le processus exécuté par le contrôleur comprend en outre :
la segmentation (311) d'un volume d'imagerie à résonance magnétique à 3 dimensions pour obtenir le volume d'imagerie à résonance magnétique segmenté à 3 dimensions ; et
l'identification (S331) du plan médian de l'organe dans le volume d'imagerie à résonance magnétique segmenté à 3 dimensions pour obtenir la représentation d'imagerie à résonance magnétique segmentée à 2 dimensions du plan médian de l'organe.

3. Contrôleur selon la revendication 2, dans lequel le processus exécuté par le contrôleur comprend en outre :
la transformation des coordonnées de la représentation d'imagerie à résonance magnétique segmentée à 2 dimensions du plan médian en coordonnées à 2 dimensions du plan médian de l'organe.

4. Contrôleur selon la revendication 1, dans lequel le plan médian de l'organe est identifié sur la base d'une entrée à l'initiative d'un utilisateur.

5. Contrôleur selon la revendication 1, dans lequel le processus exécuté par le contrôleur comprend en outre :
l'obtention de la représentation d'imagerie à résonance magnétique segmentée à 2 dimensions du plan médian de l'organe ; et
l'enregistrement de la représentation d'imagerie à résonance magnétique segmentée à 2 dimensions du plan médian de l'organe dans le plan médian ultrasonore.

6. Contrôleur selon la revendication 5, dans lequel l'enregistrement de la représentation d'imagerie à résonance magnétique segmentée à 2 dimensions du plan médian de l'organe dans le plan médian ultrasonore est réalisé en utilisant un algorithme itératif du point le plus proche.

7. Contrôleur selon la revendication 1,
dans lequel l'organe comprend une prostate.

8. Contrôleur selon la revendication 1,
dans lequel l'enregistrement de l'image du volume d'imagerie à résonance magnétique à 3 dimensions dans l'espace de suivi est réalisé en direct durant une procédure médicale interventionnelle.

9. Contrôleur selon la revendication 1, dans lequel le processus exécuté par le contrôleur comprend en outre :
la génération d'un volume ultrasonore à 3 dimensions en reconstruisant en un volume une série d'images à ultrasons à 2 dimensions spatialement suivies obtenues pendant le balayage de la sonde à ultrasons à travers l'organe.

10. Contrôleur selon la revendication 1, dans lequel le processus exécuté par le contrôleur comprend en outre :
le contrôle d'un écran pour afficher les images à ultrasons suivies en direct fusionnées avec les sections correspondantes d'images d'imagerie à résonance magnétique.

11. Contrôleur selon la revendication 1, dans lequel le processus exécuté par le contrôleur comprend en outre :
la détermination du mouvement de l'organe ; et
la re-génération, sur la base de la détermination du mouvement de l'organe, de l'enregistrement du volume d'imagerie à résonance magnétique à 3 dimensions dans l'espace de suivi sur la base des coordonnées à 2 dimensions du plan médian de l'organe, l'enregistrement de l'image du plan médian de l'organe, et la position de suivi dans l'espace de suivi de l'image à ultrasons du plan médian.

12. Procédé d'enregistrement d'une image d'imagerie à résonance magnétique (IRM) dans un espace de suivi qui comprend une sonde à ultrasons suivie et un organe capturé dans une image à ultrasons générée par la sonde à ultrasons, comprenant :
l'obtention de coordonnées à 2 dimensions d'une coupe d'un plan médian à travers une segmentation de l'organe sur la base d'une intersection d'un volume d'imagerie à résonance magnétique segmenté à 3 dimensions qui comprend la segmentation de l'organe et une représentation d'imagerie à résonance magnétique segmentée à 2 dimensions d'un plan médian de l'organe ;
le contrôle de la génération d'un volume ultrasonore à 3 dimensions ;
la segmentation du volume ultrasonore à 3 dimensions pour obtenir un volume ultrasonore à 3 dimensions segmenté ;
l'identification du plan médian de l'organe dans le volume ultrasonore segmenté à 3 dimensions pour identifier un plan médian ultrasonore ;
la génération, en utilisant une sonde à ultrasons suivie, d'une position de suivi dans l'espace de suivi d'une image à ultrasons d'un plan médian de l'organe ;
l'enregistrement, par un processeur d'un contrôleur qui comprend le processeur et une mémoire, d'une représentation ultrasonore segmentée à 2 dimensions du plan médian de l'organe fournie par le plan médian ultrasonore dans une représentation d'imagerie à résonance magnétique segmentée à 2 dimensions du plan médian de l'organe pour obtenir un enregistrement d'image du plan médian de l'organe ; et
la génération, par le processeur, d'un enregistrement du volume d'imagerie à résonance magnétique à 3 dimensions dans l'espace de suivi sur la base des coordonnées à 2 dimensions du plan médian de l'organe, l'enregistrement d'image du plan médian de l'organe, et la position de suivi dans l'espace de suivi de l'image à ultrasons du plan médian.

13. Système pour enregistrer une image d'imagerie à résonance magnétique (IRM) dans un espace de suivi qui comprend une sonde à ultrasons suivie et un organe capturé dans une image à ultrasons générée par la sonde à ultrasons, comprenant :
le contrôleur selon la revendication 1, et
une sonde à ultrasons suivie (456) ;
